# EUROPÄISCHE PATENTSCHRIFT

(11) **EP 2 741 783 B1**
(45) Veröffentlichungstag und Bekanntmachung des Hinweises auf die Patenterteilung: **22.03.2017**
(21) Anmeldenummer: 12756126.4
(22) Anmeldetag: 07.08.2012
(51) Int. Cl.: A61K 47/64, C12N 15/88, A61K 48/00

(54) **POLYANION-NANOKOMPLEXE FÜR THERAPEUTISCHE ANWENDUNGEN**
POLYANION NANOCOMPLEXES FOR THERAPEUTIC APPLICATIONS
NANOCOMPLEXES POLYANIONIQUES POUR APPLICATIONS THÉRAPEUTIQUES

(30) Priorität: 08.08.2011 DE 102011109683; 01.02.2012 DE 102012001844
(43) Veröffentlichungstag der Anmeldung: 18.06.2014
(73) Patentinhaber: Universität Regensburg, 93053 Regensburg (DE)
(72) Erfinder: GÖPFERICH, Achim, 93161 Sinzing (DE); FERSTL, Matthias, 92331 Parsberg (DE)
(74) Vertreter: Vossius & Partner Patentanwälte Rechtsanwälte mbB
(86) Internationale Anmeldenummer: PCT/EP2012/065470
(87) Internationale Veröffentlichungsnummer: WO 2013/020986

(56) Entgegenhaltungen:
- US-A1- 2006 193 825
- US-A1- 2007 185 033
- US-A1- 2010 129 460
- US-A1- 2010 311 821
- DONG-WOOK RYU ET AL: "Amphiphilic peptides with arginines and valines for the delivery of plasmid DNA", JOURNAL OF CELLULAR BIOCHEMISTRY, Bd. 112, Nr. 5, 1. Mai 2011 (2011-05-01), Seiten 1458-1466, XP055045696, ISSN: 0730-2312, DOI: 10.1002/jcb.23064
- RAYMOND S. TU ET AL: "Cooperative DNA binding and assembly by a bZip peptide-amphiphile", SOFT MATTER, Bd. 6, Nr. 5, 1. Januar 2010 (2010-01-01), Seite 1035, XP055045728, ISSN: 1744-683X, DOI: 10.1039/b922295b
- Ferstl M. ET AL: "CHARACTERIZATION OF NANOSTRUCTURED POLY-ELECTROLYTE PEPTIDE COMPLEXES (Posterbeiträge Pharmazeutische Technologie Abstract T012)", Deutsche Pharmazeutische Gesellschaft - Jahrestagung 2010 , 4. Oktober 2010 (2010-10-04), 7. Oktober 2010 (2010-10-07), XP002688155, Gefunden im Internet: URL:http://rzbl04.biblio.etc.tu-bs.de:8080 /docportal/servlets/MCRFileNodeServlet/Doc Portal_derivate_00017941/DPhG-Jahrestagung _2010.pdf [gefunden am 2012-12-03]
- MATTHIAS FERSTL ET AL: "Nanofibers Resulting from Cooperative Electrostatic and Hydrophobic Interactions between Peptides and Polyelectrolytes of Opposite Charge", LANGMUIR, Bd. 27, Nr. 23, 6. Dezember 2011 (2011-12-06), Seiten 14450-14459, XP055045725, ISSN: 0743-7463, DOI: 10.1021/la202252m

## Beschreibung

Gegenstand der vorliegenden Erfindung sind Nanokomplexe aus Polyanionen und Peptiden, welche beispielsweise für den Transport von Arzneistoffen in Zellen und deren kontrollierte Freisetzung im Organismus geeignet sind. Bestimmte strukturelle Eigenschaften eines Peptids ermöglichen es diesem mit Polyanionen kooperative elektrostatische und hydrophobe Interaktionen auszubilden. Dies führt auf molekularer Ebene zur Ausbildung von Nanokomplexen, die vorzugsweise linear sind. Kennzeichen der Komplexe ist, dass pro negativer Ladung des Polyanions eine positive Ladung eines Peptids zur Verfügung gestellt wird um den Komplex auszubilden. Dabei können beispielsweise durch die Verwendung von Nukleinsäuresequenzen mit wenigen Basenpaaren als Polyanion Nanokomplexe mit einer Größe von kleiner 10 nm hergestellt werden. Dies bedeutet eine deutliche Reduzierung der Größe beispielsweise gegenüber Polyplexen aus Polykationen und Polyanionen, bei denen es zu einer zufälligen Anordnung von Polyanionen und Polykationen kommt. Ein großer Vorteil der Komplexe ist, dass durch die geringe Größe eine Aufnahme in Zellen ermöglicht werden kann. Diese kann durch die Anbindung geeigneter biologisch aktiver Moleküle, die mit spezifischen Oberflächenstrukturen von Zellen in Wechselwirkung treten, unterstützt werden. Darüber hinaus ist es möglich Spacer, wie zum Beispiel PEG-Ketten, an die Peptide zu heften um die Zirkulationshalbwertszeit der Komplexe im Blut zu verlängern oder die biologisch aktiven Moleküle am Komplex zu verankern.

Der Transport von Nukleinsäuren, Proteinen und Peptiden in Gewebe und Zellen besitzt für die Therapie zahlreicher Erkrankungen des tierischen und menschlichen Organismus herausragende Bedeutung.

So können angeborene oder erworbene Gendefekte zum Funktionsverlust oder zu einer Überexpression von Proteinen führen. Je nach Aufgabe des betroffenen Proteins kann es zu Schädigungen des Organismus kommen und damit zur Entstehung von Krankheiten. Solche Gendefekte können durch das gezielte Einbringen von Nukleinsäuren in die Körperzellen der Patienten behandelt werden. Zu den Nukleinsäuren zählen beispielsweise alle Oligo- und Polymere der Desoxyribonucleinsäure (DNA), Ribonukleinsäure (RNA), siRNA, zyklische DNA (Plasmide), Antisenseoligonukleotide beziehungsweise Derivate all dieser Substanzen. Ursprünglich entwickelt zur Behandlung monogenetischer Erkrankungen hat sich das Anwendungsspektrum der Therapie mit Nukleinsäuren mittlerweile zusätzlich auf die Bereiche Krebs, neurologische- und vaskuläre Erkrankungen und Infektionskrankheiten ausgeweitet.

Eine wichtiger Entwicklungsschritt für die Behandlung von Krankheiten stellt das "gene silencing" dar. Dabei wird durch den gezielten Transport von siRNA in die Zellen von Patienten die Expression der für die Krankheit mitverantwortlichen Gene unterdrückt.

Die Optimierung der Transporteffizienz von Nukleinsäuren in die Zellen eines Patienten stellt dabei weiterhin eine große Herausforderung dar. Beim Transport an den Wirkungsort müssen mehrere Barrieren, wie Zellmembran oder Membranen im Zellinneren, überwunden werden. Dabei stellen sowohl eine zunehmende Größe als auch ein zunehmender Ladungszustand eines Moleküls gravierende Handicaps dar.

Nach der Applikation im tierischen oder menschlichen Organismus müssen Nukleinsäuren, zunächst ihre Zielzelle finden und nach der Kontaktaufnahme die Zellmembran überwinden, um ins Zellinnere zu gelangen. Dort schließt sich der Transport in den Zellkern oder ins Zytoplasma an. Nukleinsäuren sind Polyanionen und damit stark negativ geladene Moleküle, die in der Regel nicht durch biologische Membranen wie die Zellmembran permeieren können. Es ist daher nötig, Nukleinsäuren durch geeignete Transportstrategien selektiv und effektiv in die Zielzelle zu transportieren.

An solche Transportsysteme werden nach dem gegenwärtigen Stand der Literatur zwei wesentliche Anforderungen gestellt: Zunächst müssen sie die negative Ladung des Polyanions kompensieren und, so wird derzeit diskutiert, günstigerweise eine Umladung zu positiven Partikeln bewirken um eine elektrostatische Wechselwirkungen mit der Zellmembran über anionischen Strukturen wie Proteoglykanen auf der Zelloberfläche zu ermöglichen. Zum anderen müssen die Transportsysteme die Nukleinsäure vor einem Abbau z.B. durch Nukleasen schützen.

Bei Transportsystemen für die Klasse der Nukleinsäuren unter den Polyanionen unterscheidet man prinzipiell zwischen "viralen" und "nicht-viralen Vektoren". Der Fachmann nennt den Prozess des Nukleinsäuretransfers in Zellen dann Transduktion (mit Viren) beziehungsweise Transfektion (ohne Viren). Für andere Polyanionen gibt es derzeit nur nichtvirale Träger. Bei viralen Vektoren macht man sich zu Nutze, dass Viren evolutionär optimierte Strategien entwickelt haben um Nukleinsäuren in Zellen einzuschleusen. Obwohl virale Vektoren hocheffizient sind, haben Diskussionen um die Sicherheit bei ihrer Anwendung am Menschen die Suche nach nicht-viralen Alternativen verstärkt. Die Probleme einer viralen Therapie stellen ihr immunogenes Potential, ein mögliches onkogenes Risiko sowie generalisierte Virusinfektionen dar (T. Ratko, J. Cummings, J. Blebea, K. Matuszewski; Clinical gene therapy for nonmalignant disease. Am. J. Med. (2003); 115, 560-569).

Aufgrund dieser zahlreichen Nachteile wurden verschiedene nicht-virale Alternativen entwickelt die sich grundsätzlich auch für andere Polyanionen eignen. Dazu zählen kationische d. h. positiv geladene Substanzen wie zum Beispiel Lipide und Polymere. Hierbei können Polyanionen bedingt durch die negativen Ladungen elektrostatisch mit Kationen unter Bildung kleiner Partikel interagieren. Partikel aus Nukleinsäuren und kationischen Lipiden kennt der Fachmann unter dem Begriff Lipoplexe, Partikel aus Nukleinsäuren und Polymeren sind ihm als Polyplexe bekannt. Nachteilig an diesen Systemen ist, dass sie eine geringere Transfektionseffizienz aufweisen als virale Systeme zur Übertragung von Nukleinsäuren. D. h. deutlich weniger als 100 % der Zellen reagieren mit dem gewünschten biologischen Effekt auf die transportierte Nukleinsäure.

Komplexe, welche Polykationische Verbindungen umfassen, weisen wie Polyplexe und Lipoplexe in der Regel eine positive Überschussladung und damit eine Vielzahl von Nachteilen auf:
1. Es kann zu einer Interaktion mit negativ geladenen Zellbestandteilen kommen, was zu toxikologisch bedenklichen Effekten wie Schädigung der Zellmembran, Hemmung der metabolischen Zellaktivität oder sogar bis zum apoptotischen oder nekrotischen Zelltod führen kann.
2. Ein weiterer Nachteil liegt in derSchwieirgkeit, die Größe dieser Partikel zu kontrollieren. Der Durchmesser vieler dieser Partikel beträgt mehr als 100 Nanometer. Das erschwert die Aufnahme in die Zelle und einzelne Zellkompartimente wie den Zellkern.
3. Ein positiver Ladungsüberschuss, der dem Fachmann in Form eines positiven Zetapotentials bekannt ist, bedingt elektrostatische Interaktionen mit negativ geladenen Plasmaproteinen wie z.B. Serumalbumin, Immunglobulinen oder Fibrinogen. Diese Effekte führen teils zur Adsorption von Plasmaproteinen nebst folgender Phagozytose, teils zu einer Aggregation im Blutstrom und teils zur Anlagerung an Zellen der Blutgefässe, die ggf. nicht mit dem Bestimmungsort identisch sind.

Diesen Nachteilen von nicht-viralen Vektoren versucht man mit verschiedenen Strategien, zum Teil auch in Kombination, zu begegnen. So werden beispielsweise hydrophile nichtionische Polymere wie Polyethylengylkol (PEG) als abschirmende Komponenten eingesetzt um unspezifische Interaktionen zu verringern (US20100015050). PEGs verhindern die Adsorption von Proteinen, wie z.B. solchen des Plasmas, mit der eine Opsonisierung und damit eine verstärkte Phagozytose einhergeht. Daneben versucht man durch die Anbindung spezifischer Liganden an die Komplexe diese selektiv zu ihrem Zielort zu steuern und damit gleichzeitig Nebenwirkungen zu verhindern (US20040241855). Dabei kommen verschiedene Arten von Liganden, wie Antikörper, Wachstumsfaktoren, Vitamine, Kohlenhydrate, Integrine oder Lektine, zum Einsatz.

Eine in ihrer Bedeutung zunehmend relevante Alternative stellen Peptide für die Komplexierung von Polyanionen und insbesondere Nukleinsäuren dar. Man unterscheidet dabei zwischen verschiedenen Arten von Peptiden (A. Mann, G. Thakur, V. Shukla, M. Ganguli; Peptides in DNA delivery: current insights and future directions, Drug Discovery Today (2008), 13, 152-160). Es gibt zum einen nukleinsäureverdichtende Peptide, wie beispielsweise lineare Lysin-basierte Verbindungen. Diese reagieren aufgrund ihrer großen Anzahl von positiv geladenen Aminosäuren elektrostatisch mit den Nukleinsäuren und bilden kondensierte Partikel. Des Weiteren gibt es zellpenetrierende Peptide. Hier unterscheidet man zwischen Lysin-reichen Peptiden wie z.B. Transportan und Arginin-reichen Peptiden wie z.B. der HIV-1 trans-aktivierende transkriptionelle Aktivator (TAT). Solche Aggregate werden direkt in das Zytoplasma von Zellen unter Umgehung des endosomal/lysosomalen Kompartiments aufgenommen. Eine weitere Gruppe stellen fusogene Peptide dar. Ihr Funktionsprinzip besteht aus einer Veränderung der Konformation bedingt durch die Absenkung des pH-Wertes im Endosom. Diese Konformationsänderung ermöglicht die Wechselwirkung mit Phospholipidmembranen und macht dadurch letztendlich die Membran durchlässig. Diese Gruppe umfasst natürliche Peptide (z.B. Hämaglutinin-Untereinlieit HA-2 des Influenzavirus) sowie synthetische Peptide (z.B. GALA, KALA). Endosomolytische Peptide sind Histidin-reiche Peptide deren Prinzip auf einer Protonierung der Imidazol Gruppe von Histidin beruht welche zur folgenden Fusion und Zerstörung der Vesikelmembran führt. Daneben gibt es noch die Kernlokalisierungsignal enthaltende Peptide wie z.B. SV40 T-Antigen. Diese enthalten eine stark basische Aminosäuresequenz, typischerweise mit weniger als 12 Aminosäuren, welche durch spezifische intrazelluläre Rezeptorproteine erkannt wird und deren Transport in den Kern durch Poren in der Kernmembran ermöglichen.

Auch in der Patentliteratur (z.B. US2010/0129460 A1) werden Peptide beschrieben, welche durch die elektrostatische Interaktion mit Polyanionen und vorzugsweise Nukleinsäuren kondensierte Partikel bilden. Insgesamt bringt die Anwendung der o.g. Peptide jedoch einige Probleme mit sich:
1. Sie enthalten mehrere positive Ladungen, die alle in der Lage sind mit den negativen Ladungen der Nukleinsäuren zu reagieren. Auch die dadurch entstehende Komplexe haben einen zufälligen, unregelmäßigen Aufbau und einen positiven Ladungsüberschuss verbunden mit den oben dargestellten Problemen.
2. Ebenfalls werden aufgrund ihrer Ladung nicht nur diejenigen Verbindungen gebunden, die man in die Zellen einschleusen möchte, sondern auch solche, die natürlicherweise in der Zelle vorliegen. Folglich beobachtet man häufig, dass bei der Verwendung der o.g. Peptide zur Komplexierung von Polyanionen wie Nukleinsäuren toxische Effekte austreten, was für eine Anwendung im tierischen oder menschlichen Organismus prohibitiv ist.

Daneben besteht auch auf dem Gebiet der Peptid- und Proteinwirkstoffe erhebliches Interesse an effizienten Verabreichungssystemen. Erfolgreiche Verabreichungssystemen können beispielsweise die Pharmakokinetik der Wirkstoffe positiv beeinflussen, den Wirkstoffabbau minimieren, schädliche systemische Nebeneffekte verhindern, die Bioverfügbarkeit des Wirkstoffs erhöhen und/oder erlauben es, die Verabreichungsfrequenz senken. Allerdings konnten für Peptide und Proteine viele dieser Verabreichungsstrategien noch nicht zufriedenstellend umgesetzt werden, da sie besondere Anforderungen an geeignete Systeme stellen. Ein Ansatz besteht in der Verwendung eines Hydrogels als Matrix für den zu verabreichenden Wirkstoff, wobei aber häufig das Abgabeprofil des Wirkstoffs nicht optimal ist, und eine schnelle Freisetzung nicht verhindert werden kann.

Daher besteht nach wie vor Bedarf an Verabreichungssystemen, mit deren Hilfe Wirkstoffe wie beispielsweise Nukleinsäuren und ihre Oligomere und Polymere, Peptide oder Proteine auf effiziente Weise dem Organismus zugeführt und dort vorzugsweise kontrolliert freigesetzt werden können.

Bei der Untersuchung von Interaktionen zwischen Polyanionen und Peptiden hat sich gezeigt, dass die Komplexierung von Polyanionen durch bestimmte Peptide zur Bildung von definierten Nanostrukturen führt. Gegenstand der Erfindung sind Komplexe aus Polyanionen mit Peptiden, die bestimmte Eigenschaften aufweisen. Insbesondere werden im Rahmen der Erfindung Nanokomplexe bereit gestellt, bestehend aus einem Polyanion und kationischen amphiphilen Peptidmolekülen in äquimolarem Verhältnis zu den negativen Ladungen des Polyanions. Durch die Kombination des Polyanions mit derartigen Peptidmoleküle werden Komplexe gebildet, die durch kooperative molekulare Wechselwirkungen zusammengehalten werden. Darüber hinaus wird ein Komplexmaterial bereit gestellt, in dem diese Komplexe mit einer biologisch aktiven Einheit verknüpft sind, sowie Arzneimittel, die die Nanokomplexe oder Komplexmaterialien enthalten.

Der Begriff des Polyanions bezeichnet auf dem Fachgebiet generell Moleküle, die mehrere negativ geladene Gruppen, z.B. Carbonsäureanionen, enthalten. Bevorzugt kommen im Rahmen der Erfindung solche Polyanionen zu Einsatz, die bei einem physiologischen pH-Wert, z.B bei einem pH-Wert innerhalb des Bereichs von pH 7,0 bis 7,5 in wässrigen Lösungen, eine Vielzahl negativ geladener Gruppen aufweisen. Insbesondere geeignet sind dabei Polymere, die polymerisierte Untereinheiten mit jeweils einer oder mehreren anionischen Gruppen enthalten. Erfindungsgemäß besonders bevorzugte Polyanionen sind damit zum einen Polysaccharide, wie beispielsweise Alginat, Hyaluronat, Carboxymethylcellulose, Carrageenan oder Xanthan. Ebenfalls bevorzugt können Nukleinsäuren, wie beispielsweise Oligomere oder Polymere einzelsträngiger oder doppelsträngiger Ribonukleinsäure (RNA) oder Desoxyribonukleinsäure (DNA) zum Einsatz kommen. Ein Polyanion im Sinne der Erfindung kann mehr als 10.000 negative Ladungen tragen. Plasmide sind ein Beispiel für solche Moleküle. Bei Alginat, welches ein anderes typisches Polyanion ist, findet man ca. 1.000 negative Ladungen. Auch small interfering RNA (siRNA) kann als Polyanion eingesetzt werden, als Einzel- und bevorzugt als Doppelstrang, beispielsweise im Einzelstrang mit 21 Ladungen, im Doppelstrang mit 42 Ladungen, die von der Phosphatgruppe stammen. Schließlich sind auch Antisensenukleotide als ein Beispiel geegineter Polyanionen zu nennen. Je nach pH können die Basenpaare von DNA und RNA ggf zusätzliche positive Ladungen generieren. Auch Derivate dieser beispielhaft aufgeführten Substanzen sind erfindungsgemäß geeignet, soweit die Derivatisierung den polyanionischen Charakter der Substanzen nicht beeinträchtigt.

Im Sinne der Erfindung finden insbesondere kationische Peptide Verwendung, die je Molekül weniger als 5 positive Ladungen tragen. Bevorzugt sind solche, die 2 und ganz besonders bevorzugt solche, die 1 positive Ladung tragen. Ladungsträger können eine entsprechende Anzahl an natürlichen oder nicht-natürlichen Aminosäuren sein, die entweder permanent oder pH-abhängig eine positive Ladung tragen. Beispiele für geeignete natürliche Aminosäuren sind Arginin oder Lysin. Im Sinne der Erfindung könnte auch das Aminoende eines Peptids allein Ladungsträger sein. Soweit die Ladung in Abhängigkeit vom pH-Wert auftritt, werden Peptide bevorzugt, die je Molekül bei einem physiologischen pH-Wert, z.B bei einem pH-Wert innerhalb des Bereichs von all 7,0 bis 7,5 in wässrigen Lösungen, weniger als 5, insbesondere 2 und ganz besonders bevorzugt 1 positive Ladungen tragen. Peptide im Sinne der Erfindung können auch molekulare Bausteine enthalten, die aufgrund ihrer Struktur zwar keine Aminosäuren sind, aber protonierbare oder permanent positiv geladener funktionelle Gruppen enthalten. In jedem Fall enthalten die Peptide mindestens 1 natürliche oder nicht-natürliche Aminosäure. Bevorzugt enthalten kationische Peptide zur erfindungsgemäßen Verwendung bis zu 20 Aminosäuren. Die erfindungsgemäßen Komplexe können, je nach Bedarf und gewünschten Eigenschaften des Komplexes, einen Typ oder ein Gemisch von zwei oder mehr Typen der kationischen Polymere enthalten.

Die Peptide orientieren sich in den erfindungsgemäßen Komplexen so zum Polyanion, dass sie über die Wechselwirkung zwischen positiver Ladung des Peptids mit einer negativen Ladung des Polyanions an dieses binden. Komplexe im Sinne der Erfindung sind durch die ionische Wechselwirkung zwischen Polyanion und Peptid stabilisiert. Eine zusätzliche Stabilisierung wird durch weitere molekulare Wechselwirkungen zwischen Peptidmoleküle erreicht, wodurch es zu einer kooperativen Wechselwirkung kommt. Die kooperativen molekularen Wechselwirkungen, die für den Zusammenhalt der erfindungsgemäßen Nanokomplexe sorgen, beinhalten damit sowohl die ionischen Wechselwirkungen zwischen Polyanion und kationischem Peptid, als auch zusätzliche kooperative Wechselwirkungen zwischen den kationischen Peptidmolekülen. Solche Wechselwirkungen können beispielsweise Wasserstoffbrückenbindungen, oder van der Waals Wechselwirkungen sein. Weitere Mechanismen sind dem Fachmann aus der Literatur der molekularen Wechselwirkungen oder der Protein/ Protein Wechselwirkungen bekannt. Ganz besonders bevorzugt sind Peptide, deren einzelne Moleküle untereinander hydrophobe Wechselwirkungen ausbilden. Dazu eignen sich hydrophobe natürliche oder nicht-natürliche Aminosäuren oder auch andere hydrophobe molekulare Bausteine. Diese enthalten beispielsweise gesättigte oder ungesättigte Kohlenwasserstoffe oder Aromaten, wie beispielsweise einen oder mehrere Phenylreste, einen oder mehrere Naphtylreste oder einen oder mehrere Pyridinylrest in ihrer molekularen Struktur. Dabei sollten die Bezeichnungen "Phenyl" usw. nicht so verstanden werden, dass nur einwertige Reste dieses Typs für eine entsprechende Wechselwirkung sorgen können, sondern dass derartige Aromaten auch als zwei oder höherwertige Reste innerhalb des Peptids vorliegen können.Beispiele für hydrophobe natürliche Aminosäuren mit aromatischen Ringen sind Tryptophan, Tyrosin oder Phenylalanin.

Kationische Peptide, die sich im Sinne der Erfindung als ganz besonders für eine Komplexbildung eignen, sind amphiphil. Aufgrund ihrer positiven Ladung für die ionische Wechselwirkung mit einer negativen Ladung des Polyanions und der hydrophoben Struktur zur Wechselwirkung mit anderen Peptidmolekülen reichern sich solche Peptide gerne in Grenzflächen an. Diese Peptide haben in der Regel eine oder mehrere der folgenden Fähigkeiten: Herabsetzen von Grenzflächenspannungen, Senken der Oberflächenspannung von vorzugsweise wässrigen Flüssigkeiten, Bildung von Mizellen, Bildung von inversen Mizellen. Beispiele für solche Peptide kennt der Fachmann unter dem Namen Cetrorelix, Degarelix, Ozarelix oder Abarelix.

Die Komplexe aus Polyanion und Peptid entstehen vorzugsweise spontan bei Mischung zweier Lösungen. Dazu werden Polyanion und Peptid in jeweils einem Lösungsmittel, z.B. Wasser, gelöst in definierter Konzentration und definiertem Volumen zusammengegeben. Dies kann außerhalb des menschlichen oder tierischen Organismus erfolgen oder in situ, d.h. im Rahmen der Applikation beispielsweise durch Injektion wie mit einer Zweikammerspritze. Optional kann es sein, dass es erforderlich ist, physikochemische Parameter wie pH, Ionenstärke oder osmotischen Druck vor oder nach dem Mischen einzustellen.

Es hat sich als besonders vorteilhaft erwiesen, bei der Herstellung der Komplexe das Verhältnis zwischen den Polyanionen und den kationischen Peptidmolekülen so einzustellen, dass sich im Komplex ein äquimolares Verhältnis der kationischen Peptidmoleküle zu den vorhandenen negativen Ladungen des Polyanions ergibt Insbesondere ist es wie vorstehend dargelegt bevorzugt, Peptide mit genau einer positiven Ladung einzusetzen, so dass für jedes Mol negativ geladener Gruppen des Polyanions ein Mol kationisches Peptid mit einer Ladung vorliegt, und der Ladungsausgleich auf diese Art und Weise hergestellt wird.

Die Stabilität der Komplexe lässt sich über die Bindungskonstante K ausdrücken die beispielsweise über isothermale Titrationskalorimetrie (ITC) bestimmt werden kann. Komplexe im Sinne der Erfindung haben in der Regel K-Werte, die größer als 10.000 Mol⁻¹ sind. Bevorzugt sind solche Komplexe mit K-Werten größer als 100.000 Mol⁻¹ und ganz besonders bevorzugt solche mit K-Werten über 1.000.000 Mol⁻¹, wobei die entsprechenden Werte bequem bei Raumtemperatur, d.h. 25 °C, bestimmt werden können.

In wässriger Lösung kann also die positive Ladung des Peptids elektrostatisch mit der negativen Ladung eines Polyanions, wie zum Beispiel den Phosphatgruppen einer Nukleinsäure, interagieren. Durch die Interaktion weiterer Peptidmoleküle mit dem Polyanion stehen sich benachbarte Peptidmolekülen so nahe, dass dies zur Ausbildung von molekularen Wechselwirkungen wie zum Beispiel hydrophoben Wechselwirkungen untereinander führt. Diese zusätzlichen Wechselwirkungen führen zu einer Stabilisierung der gebildeten Komplexe. Um das Polyanion bildet sich durch die kooperative elektrostatische und beispielsweise hydrophobe Wechselwirkung eine ,Peptidhülle' aus, welche die Ladungen des Polyanions nach außen hin abschirmt. Auf diese Weise kann beispielsweise durch die Auswahl der kationischen Polymere die Form der Komplexe kontrolliert werden, und es können Komplexe mit einer definierten Form bereit gestellt werden, deren Bestandteile eine regelmäßige Anordnung aufweisen.

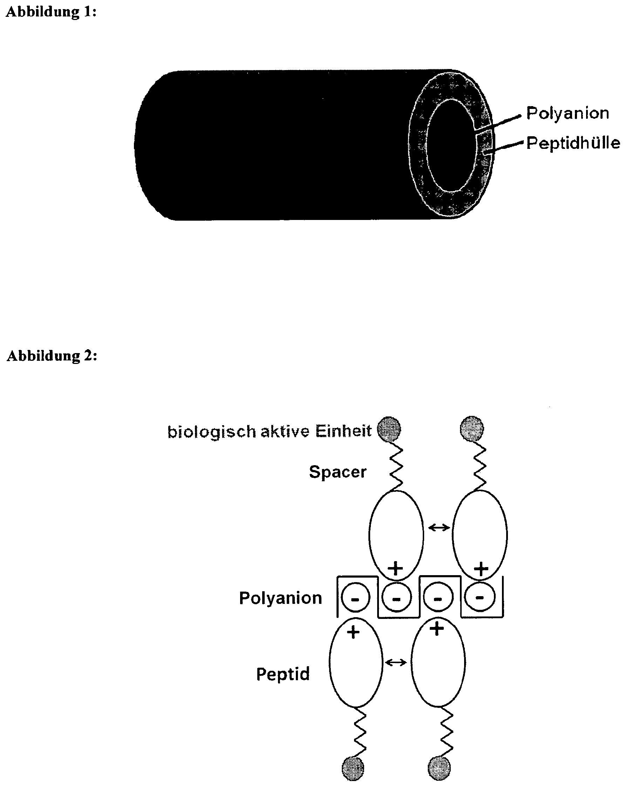

Ein Beispiel eines solchen Komplexes ist in Abb. 1 schematisch in dreidimensionaler Darstellung gezeigt, wobei der Fachmann verstehen wird, dass die Seitenflächen des als Zylinder dargestellten Komplexes einen Schnitt durch eine typische Komplexstruktur wiedergeben. Auch Abbildung 2 verdeutlicht in zweidimensionaler Form beispielhaft den Aufbau eines entsprechenden Komplexes, bei dem sich einfach geladene Peptide um ein Polyanion anordnen. Bei Verwendung eines Polyanions mit linearer Struktur können so nach einer bevorzugten Ausführungsform der Erfindung lineare Komplexe erzeugt werden.. Die Länge der vorzugsweise linearen Komplexe wird bestimmt durch die Anzahl der Monomeren des Polyanions, wie zum Beispiel die Anzahl der Basen einer Nukleinsäuren, und beträgt zwischen 1 und mehr als 100 nm. Je mehr Monomere die Kette eines Polyanions enthält desto ,länger' wird der entsprechende Komplex. Der Durchmesser der vorzugsweise linearen Komplexe lässt sich dagegen über die räumliche Struktur der Aminosäuresequenz des Peptids steuern. Der Durchmesser liegt in der Regel bei 1 bis 100 Nanometern. Vorzugsweise ist er kleiner als 50 Nanometer. Ganz besonders bevorzugt sind Durchmesser kleiner als 20 Nanometer. Verzweigte Polyanionen können insgesamt auch zu verzweigten Komplexen führen.

Das Verhältnis von angelagerten Peptidmolekülen pro ladungstragendem Monomer des Polyanions, welches beispielsweise über isothermale Titrationskalorimetrie bestimmt werden kann, liegt zwischen 0,1 und 10. Bevorzugt liegt es zwischen 0,2 und 5 und ganz besonders bevorzugt zwischen 0,5 und 2. Ein Verhältnis zwischen 0,9 und 1,1 ist als weiterhin bevorzugt anzusehen, wobei insbesondere ein Verhältnis von 1 am stärksten bevorzug ist.

Die Komplexe können als solche angewendet oder weiter unter Bildung eines Komplexmaterials modifiziert werden (siehe Abbildung 2). So können an die Peptide vor oder nach der Bildung des Komplexes Spacer (Abstandshalter) angehängt werden, z.B. durch kovalente Verknüpfung durch auf dem Fachgebiet bekannte Reaktionen. Solche Spacer können als abschirmende Komponente zum Schutz gegen Enzyme, oder wie oben dargestellt gegen Adsorption von Proteinen und damit einhergehender Opsonisierung und sich anschließender Phagozytose eingesetzt werden. Der Spacer kann auch je nach eigener Ladung die Ladung des entstandenen Komplexes neutral oder negativ werden lassen. Der Spacer kann je nach Bedarf eine charakteristische chemische Struktur aufweisen. Die Zahl der Spacer kann mit der Zahl der im Komplex vorhandenen kationischen Peptidmoleküle übereinstimmen, kann aber auch darüber liegen (z.B. bei Einsatz von mehr als einem Spacer je Peptidmolekül), oder geringe sein. Einerseits eignen sich Moleküle, die dem Fachmann als sog. Linker bekannt sind, andererseits solche aus der Gruppe der Polymere. Solche Polymere können z.B. eine Molmasse bis 50.000 Dalton aufweisen. Besonders eignen sich Polymere mit einer Molmasse bis 5.000 Dalton. Ganz besonders bevorzugt sind solche mit einer Molmasse bis 1.000 Dalton. Bevorzugt werden Materialen verwendet, die bekannt dafür sind Stealth-Eigenschaften zu vermitteln, wie hydrophile anionische oder nichtionische Polymere. Beispiele sind Polyethylenglykole, Polysaccharide wie Polysialinsäure, Polyoxazoline und Polyglycerol.

Die erfindungsgemäßen Komplexe können darüber hinaus mit biologisch aktiven Einheiten bereit gestellt werden. So kann beispielsweise der Transport der Komplexe zu ihrem Ziel, d.h. beispielsweise einem bestimmten Gewebe, einer bestimmten Zelle oder einer intrazellulären Organelle, unterstützt werden. Damit können gleichzeitig Nebenwirkungen an gesunden oder Nicht-Zielzellen verhindert werden. Eine solche biologisch aktive Einheit kann bereits durch das kationische Peptid selbst, d.h. ohne Spacer, bereit gestellt werden wenn es selektiv, beispielsweise über einen Rezeptor, an das Ziel bindet. Alternativ dazu kann ein Komplexmaterial durch Modifikation der erfindungsgemäßen Nanokomplexe gebildet werden, indem diese, bevorzugt kovalent, mit einer biologisch aktiven Einheit verknüpft werden. Diese Einheit kann beispielsweise direkt oder über einen Spacer an das Peptid gebunden sein. Die biologisch aktive Einheit kann in diesem Fall vor oder nach der Bildung des Komplexes mit dem Spacer oder Peptid verknüpft werden.

Beispiele für derartige biologisch aktive Einheiten sind Liganden für verschiedene Rezeptoren der Zelloberfläche, Antiköper und andere Moleküle, die dem Fachmann für Zwecke der Zell- oder Organellerkennung bekannt sind. Je nach Zielgewebe können sowohl niedermolekulare Substanzen wie Zucker (Galactose) oder Vitamine (Folsäure) aber auch Peptide und Makromoleküle wie Proteine (Antikörper) oder Polysaccharide als Liganden eingesetzt werden. Für die selektive Ansteuerung von Tumoren bieten sich Rezeptoren von Wachstumsfaktoren wie beispielsweise der epidermal growth factor (EGF) an, die in Tumorzellen bedingt durch Ihre metabolische Aktivität oder durch Mutation in erhöhter Anzahl auf der Zelloberfläche vorliegen. Weitere Zielstrukturen für Liganden können G-Protein gekoppelte Rezeptoren (GPCR) sein. Ebenso gelten virale Rezeptoren als mögliche Zielstrukturen wie beispielsweise CD4 bei HIV, PVR bei Polioviren oder Sialinsäure bei Influenzaviren. Ein weitere Beispiel sind Rezeptoren aus der Gruppe der Integrine. Komplexe im Sinne der Erfindung bestehen damit mindestens aus Polyanion und einer entsprechenden Anzahl Peptidmolekülen. Die Anbindung von Spacern und/oder einer biologisch aktiven Einheit ist optional möglich.

Die erfindungsgemäßen Komplexe und die modifizietten Komplexmaterialien eignen sich zur Anwendung in einem Verfahren zur therapeutischen Behandlung des menschlichen oder tierischen Körpers, d.h. vornehmlich zur Verwendung als Arzneimittel. Dabei umfasst die Anwendung der Erfindung insbesondere die Verwendung der Komplexe zum Transfer von Arzneistoffen, z.B. von Nukleinsäuren in Zellen. Besonders gut eignen sich Komplexe mit einer Größe von weniger als 100 Nanometern für den Transfer von Arzneistoffen in Gewebe, Zellen und deren intrazellulären Kompartimente und Organellen. Arzneistoffen sind in diesem Zusammenhang Substanzen, die einen biologischen Effekt herbeiführen. Vorzugsweise kann dieser ein pharmakologischer Effekt sein. Arzneistoff kann einerseits das Polyanion sein, beispielsweise wenn es aus der Gruppe der Nukleinsäuren stammt. Andererseits ist es aber auch möglich, dass das Peptid ein Arzneistoff ist. Eine weitere Möglichkeit besteht darin, dass sowohl das Polyanion als auch das Peptid Arzneistoffe sind. In diesem Zusammenhang ergeben sich interessante Kombinationen von Substanzen die synergistisch wirken können. So zum Beispiel in der Tumortherapie. Hier könnte beispielsweise das siRNA-silencing in Kombination mit Peptiden die zytostatisches Potenzial haben, deutliche Synergismen erzielen. Über die einstellbare geringe Größe der Komplexe ist es darüber hinaus möglich die Komplexe nach intravasaler Applikation im Tumorgewebe anzureichern, was dem Fachmann unter anderem als ,enhanced permeability and retention effect' bekannt ist.

Die Nanokomplexe bzw. Komplexmaterialien können aber auch zur kontrollierten Freisetzung von Arzneistoffen nach parenteraler, insbesondere nicht-intravasaler d.h. beispielsweise subkutaner, intramuskulärer, intraokularer oder sonstiger lokaler Applikation dienen. Der Arzneistoff kann dabei sowohl lokal, d.h. unmittelbar am Applikationsort, als auch systemisch, d.h. nach Verteilung im menschlichen oder tierischen Organismus, wirksam sein. Beilspielsweise können die Komplexe im Sinne der Erfindung zur Steuerung der Freigabe von therapeutisch wirksamen Peptiden oder Nukleinsäuren genutzt werden. Dabei können auch Komplexe zum Einsatz kommen, die eine Länge von mehr als 100 nm aufweisen.

Die Nanokomplexe können einzeln vorliegen oder sich zu größeren Aggregaten zusammenschließen. In diesem Zusammenhang ist es möglich, dass die Aggregatlänge wächst, ohne dass sich der Durchmesser ändert. Darüber hinaus können auch unregelmäßige Aggregate entstehen, bis hin zu Systemen die einen gelartigen Charakter besitzen. Ihre Funktionalität insbesondere für die Kontrolle der Freisetzung des Arzneistoffs bleibt dabei aber erhalten.

Therapeutische Anwendungen der Nanokomplexe bzw. Komplexmaterialien, insbesondere bei Verwendung von Nukleinsäuren als Polyanion, umfassen beispielsweise die Therapie monogenetischer Erkrankungen, aber auch von Krebs, neurologischen und vaskulären Erkrankungen sowie Infektionskrankheiten.

Arzneimittel, die im Rahmen der Erfindung bereit gestellt werden, können aus den Nanokomplexen oder Komplexmaterialien als solchen bestehen, oder die Nanokomplexe oder Komplexmaterialien zusammen mit auf dem Fachgebiet bekannten Hilfsstoffen enthalten, beispielsweise ausgewählt aus Verdünnungsmitteln einschließlich Lösungsmitteln, Substanzen zum Einstellen gewünschter Salzkonzentrationen und/oder eines gewünschten pH-Werts und Konservierungsmitteln enthalten. Geeignete, pharmazeutisch verträgliche Hilfstoffe die bei der Herstellung der erfindungsgemäßen Pharmazeutischen Zusammensetzungen Verwendung finden können sind dem Fachmann bekannt und sind beispielsweise in Remingion's Pharmaceutical Sciences, 15th Ed., Mack Publishing Co., New Jersey (1991) beschrieben.

Geeignete pharmazeutische Darreichungsformen können durch im Fachgebiet etablierte, pharmazeutisch technische Herstellungsverfahren bereitgestellt werden. Dabei kann eine Vielzahl der dem Fachmann bekannten Darreichungsformen für die Formulierung der Nanokomplexe und Komplexmaterialien geeignet sein. Beispiele für pharmazeutische Darreichungsformen sind feste Arzneiformen (z.B. Lyophilisate, Pulver, Granulate, Tabletten, Dragees, Kapseln Implantate etc.), halbfeste Arzneiformen (z.B. Salben, Gele, Pflaster, Emulsionen, Suspensionen etc.), flüssige Arzneiformen (Lösungen, Injektions- u. Infusionszubereitungen, Ocularia etc.) und therapeutische Systeme (z.B. orale osmotische therapeutische Systeme, Transdermalsysteme, Inserte etc). Beispielhaft sei an dieser Stelle auf Pharmaceutical Dosage Forms and Drug Delivery Systems, H.C. Ansel et al., eds., 7th ed., Lippincott, Williams, & Wilkins (1999) verwiesen.

In Abhängigkeit vom individuell verwendeten Nanokomplex bzw. Komplexmaterial und der ausgewählten Darreichungsform kann die Verabreichung auf eine Vielzahl, dem Fachmann bekannter Wege erfolgen. Beispiele hierfür sind perorale, inhalative, topische, intravenöse, intraperitonale, subkutane und intramuskuläre Verabreichung.

Bevorzugt werden die Nanokomplexe und Komplexmaterialien als zur parenteralen Applikation geeignete Lösungen oder Suspensionen formuliert. Dabei kann ein erfindungsgemäßes Arzneimittel in vorteilhafter Weise formuliert werden, indem Polyanionen mit Polykationen, ggf. unter Zusatz weiterer Hilfsstoffe, gemischt werden. Als ebenfalls bevorzugte Ausführungsform ist weiterhin anzusehen, dass die zur parenteralen Applikation geeigneten Darreichungsformen zuvor lyophilisiert werden. Eine Lyophilisation kann vor allem sinnvoll sein, um eine ausreichende Lagerstabilität der zu verabreichenden Darreichungsform zu gewährleisten. Geeignete Verfahren zur Gefriertrocknung sowie konventionell eingesetzte Zusatzstoffe (z.B. kryoprotektive Zusatzstoffe,wie z.B. Laktose, Mannitol etc.) sind dem Fachmann bekannt. Im Falle der Gefriertrocknung kann z.B. eine zu verabreichende Lösung kurz/unmittelbar vor Gebrauch durch Aufnahme des Lyophilisats in geeigneten Solventien, wie z.B. Wasser zur Injektion, isotoner Kochsalzlösung, Ringer-Lösung etc, hergestellt werden.

In Betracht kommt auch, die erfindungsgemäßen Nanokomplexe oder Komplexmaterialien unmittelbar vor oder während der Applikation durch Mischen der Bestandteile herzustellen, oder sie sogar im Körper, z.B. nach paralleler oder zeitlich kurz aufeinander folgender Verabreichung der Bestandteile, entstehen zu lassen.

### Beispiele

### Beispiel 1 (nicht erfindungsgemäß)

Somatostatin wird in einer Konzentration von 0.225 mg/ml in Wasser pH= 6 gelöst. Doppelsträngige RNA (Strang 1: r(GCAAGCUGACCCUGAAGUUCA)dT, Strang 2: r(GAACUUCAGGGUCAGCUUGCC)dT) bestehend aus 22 Basenpaaren in einer Konzentration von 0,04 mg/ml wird ebenfalls in Wasser gelöst. Die entsprechenden Mengen werden dabei so berechnet, dass ein Ladungsausgleich zwischen positiver und negativer Ladung erreicht wird. Dazu wird die Konzentration der Nukleinsäurelösung durch die Molmasse der doppelsträngigen RNA und durch 2 (positive Ladungen pro Somatostatinmolekül) geteilt und anschließend mit 44 (Menge an negativen Ladungen pro Mol RNA) und der Molmasse des Somatostatins multipliziert, um die Konzentration der Somatostatinlösung zu erhalten. Es wird 1 ml der Nukleinsäurelösung in einem Eppendorf Tube vorgelegt und 1 ml der Peptid Lösung langsam zugetropft. Danach wird die Lösung auf einem Vortexmischer bei Raumtemperatur durchmischt. Anschließend wird die Lösung bei Raumtemperatur für 1 h auf einen Laborschüttler gestellt um die Ausbildung eines Gleichgewichts zwischen Komplex und den freien Komponenten zu gewährleisten.

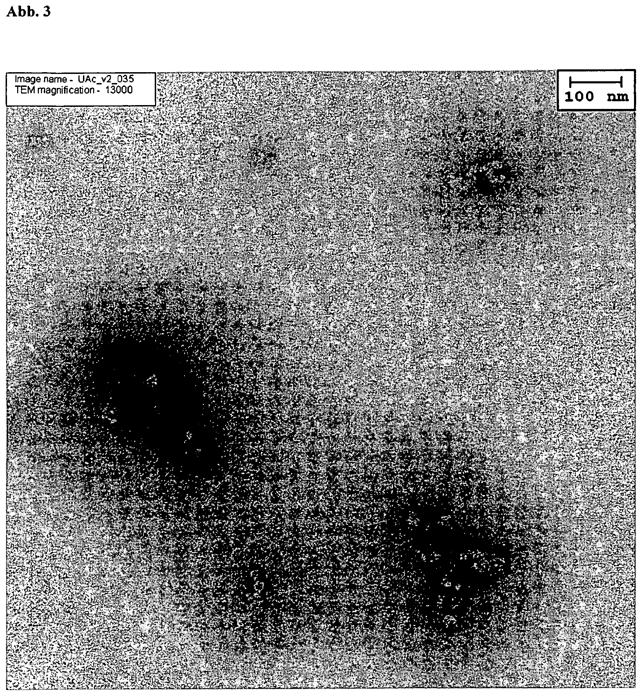

Abb. 3 zeigt eine transmissionsciektronenmikroskopische Aufnahme der Nanokomplexe gebildet aus Somatostatin und doppelsträngiger RNA aus Beispiel 1.

### Beispiel 2 (nicht erfindungsgemäß)

Die Herstellung der Ausgangslösungen erlolgt analog zu Beispiel 1. Jedoch wird nun zunächst die Peptidlösung vorgelegt und anschließend die Nukleinsäurelösung langsam zugetropft. Anschließend wird die Lösung wiederum durchmischt.

### Beispiel 3

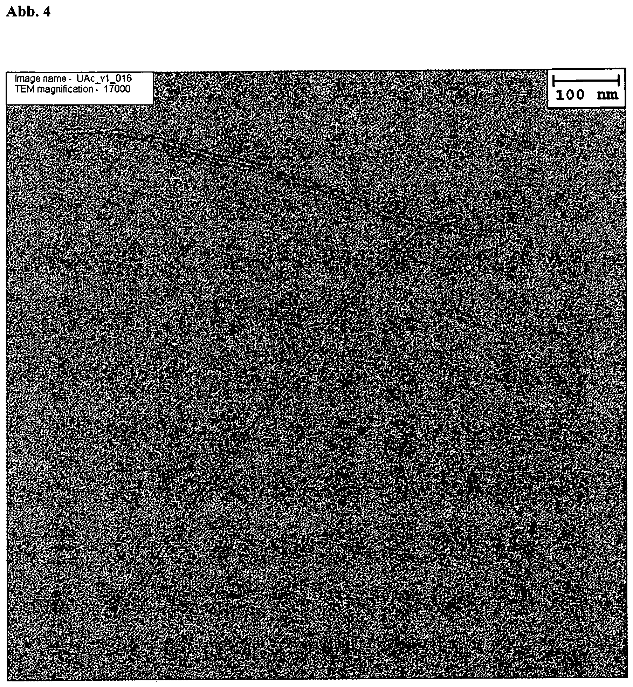

In ähnlicher Weise wie in Beispiel 1, außer dass als kationisches Polymer Ozarelix in einer entsprechenden Menge unter Berücksichtigung einer positiven Ladung pro Peptidmolekül eingesetzt wird, werden durch die Verwendung der doppelsträngigen RNA Nanokomplexe hergestellt. Abb. 4 zeigt die transmissionselektronenmikroskopische Aufnahme von Nanokomplexen gebildet aus Ozarelix und doppelsträngiger RNA.

### Beispiel 4

Cetrorelix wird in einer Konzentration von 0,00175 mmol/L in Wasser gelöst. Doppelsträngige DNA (Strang 1: ATGAACTTCAGGGTCAG TTGC, Strang 2: TACTTGAAGTCCCAGTCGAACG) bestehend aus 22 Basenpaaren in einer Konzentration von 0,0019 mmol/L wird in Puffer bestehend aus 10 mmol/L Tris, 100mmol/L NaCl und 1 mmol/L EDTA gelöst. Beide Lösungen wurden unmittelbar vor der Messung unter leichtem Rühren entgast. 1,435 ml doppelsträngige DNA werden in der Reaktionszelle der ITC vorgelegt und langsam mit 0,3 ml der Cetrorelix-Lösung titriert. Hierbei werden jeweils 10µl Cetrorelix-Lösung über 10 Sekunden zugetopft und anschließend ein Intervall von 180 Sekunden eingehalten, bis der nächste Tropfen zugegeben wird, um die gesamte freiwerdende Wärmemenge zu erfassen. Der Versuch wird bei 25±1 °C durchgeführt.

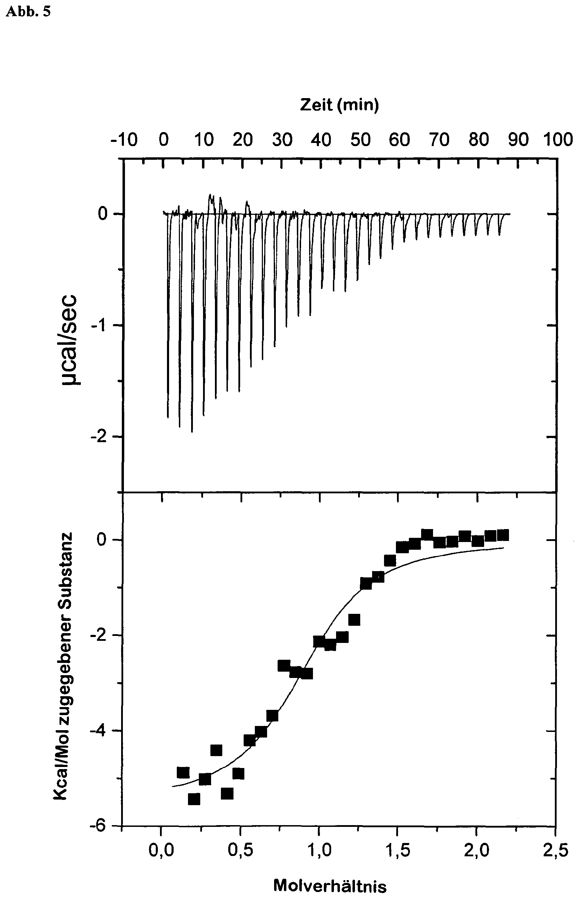

Die Daten werden mit MicroCal Origin 5.0 (MicroCal) Software ausgewertet. Hierzu wird ein nichtlineares Fitting angewendet. Durch Verwendung des "one-set of binding sites"-Model können die thermodynamischen Konstanten Standardreaktionsenthalpie (ΔH), Bindungskonstante (K), Standardrealctionsentropie (ΔS) und das stöchiometrische Verhältnis der Reaktion (n) berechnet werden. Der entstehende Komplex hat eine hohe Bindungskonstante im Bereich von 10⁵, was eine starke Bindung mit elektrostatischen und hydrophoben Wechselwirkungen anzeigt. Der negative Wert für ΔH und der positive Wert für AS sind jeweils energetisch günstig, wobei ΔS die hydrophoben Wechselwirkungen repräsentiert und ΔH ein Maß für die elektrostatischen Wechselwirkungen und Wasserstoffbrückenbindungen ist. Durch das stöchiometrische Verhältnis von 0,9247 wird gezeigt, dass jeweils ein positiv geladenes Peptid an eine negative Ladung der doppelsträngigen DNA bindet. Die folgende Tabelle zeigt die thermodynamischen Parameter des DNA-Cetrorelix-Komplexes. Abb. 5 zeigt das mittels ITC bestimmt Thermogramm der Bindung von DNA und Cefirorelix.

**Tabelle 1: Thermodynamische Parameter des DNA-Cetrorelix-Komplexes**

| | |
|---|---|
| n | 0,9247 (±0,0330) |
| K (M⁻¹) | 2,312x10⁵ |
| | (±6,696x10⁴) |
| ΔH (cal*mol⁻¹) | -5515 (±277,9) |
| ΔS (cal*mol⁻¹*K⁻¹) | 6,044 |

## Patentansprüche

1. Geometrisch definierte Nanokomplexe, bestehend aus einem Polyanion und kationischen amphiphilen Peptidmolekülen in äquimolarem Verhältnis zu den vorhandenen negativen Ladungen, **dadurch gekennzeichnet, dass** sie durch kooperative molekulare Wechselwirkungen zusammengehalten werden, und dass die Peptidmoleküle jeweils 1 positive Ladung tragen.

2. Nanokomplexe nach Anspruch 1, **dadurch gekennzeichnet, dass** es sich bei dem Polyanion, den Peptidmolekülen oder beiden um einen Arzneistoff für die Anwendung im tierischen oder menschlichen Organismus handelt.

3. Nanokomplexe nach Anspruch 1 oder 2, **dadurch gekennzeichnet, dass** die Peptidmoleküle jeweils bis zu 20 Aminosäuren enthalten.

4. Nanokomplexe nach einem der Ansprüche 1 bis 3, **dadurch gekennzeichnet, dass** das Polyanion ausgewählt ist aus der Gruppe bestehend aus Polysacchariden, Nukleinsäuren und deren Derivate.

5. Nanokomplexe nach einem der Ansprüche 1 bis 4, **dadurch gekennzeichnet, dass** sie eine lineare Form aufweisen.

6. Komplexmaterial, in dem die Nanokomplexe nach einem der Ansprüche 1 bis 5 mit einer biologisch. aktiven Einheit für die selektive Erkennung von Geweben, Zellen oder Organellen verknüpft sind.

7. Komplexmaterial nach Anspruch 6, **dadurch gekennzeichnet, dass** die biologisch aktive Einheit jeweils über einen Spacer mit dem Nanokomplex verknüpft ist.

8. Nanokomplexe oder Komplexmaterial nach einem der Ansprüche 1 bis 7 zur Verwendung in der Therapie.

9. Nanokomplexe oder Komplexmaterial nach einem der Ansprüche 1 bis 7 zur Verwendung bei der kontrollierten Freisetzung von Arzneistoffen nach parenteraler Applikation.

10. Nanokomplexe oder Komplexmaterial nach einem der Ansprüche 1 bis 7 zur Verwendung beim Transport von Arzneistoffen in Zellen.

11. Nanokomplexe oder Komplexmaterial nach einem der Ansprüche 1 bis 7 zur Verwendung beim Transport von Nukleinsäuren in Zellen.

12. Nanokomplexe oder Komplexmaterial nach einem der Ansprüche 8 bis 11, wobei die Verwendung auf die Therapie einer Erkrankung, ausgewählt aus monogenetischen Erkrankungen, Krebs, neurologischen Erkrankungen, vaskulären Erkrankungen und Infektionskrankheiten ausgerichtet ist.

13. Verfahren zur Formulierung eines Arzneimittels, das das Mischen von Polyanionen mit Peptidmolekülen unter Bildung der Nanokomplexe nach einem der Ansprüche 1 bis 5 umfasst.

## Claims

1. Geometrically defined nanocomplexes consisting of a polyanion and cationic amphiphilic peptide molecules in an equimolar ratio to the negative charges present, **characterized in that** they are held together by means of cooperative molecular interactions and **in that** the peptide molecules carry 1 positive charge each.

2. Nanocomplexes according to claim 1, **characterized in that** the polyanion, the peptide molecules or both are a drug for application in the animal or human organism.

3. Nanocomplexes according to claim 1 or 2, **characterized in that** the peptide molecules contain up to 20 amino acids each.

4. Nanocomplexes according to any one of claims 1 to 3, **characterized in that** the polyanion is selected from the group consisting of polysaccharides, nucleic acids and derivatives thereof.

5. Nanocomplexes according to any one of claims 1 to 4, **characterized in that** they have a linear shape.

6. Complex material in which the nanocomplexes according to any one of claims 1 to 5 are linked to a biologically active moiety for the selective recognition of tissues, cells or organelles.

7. Complex material according to claim 6, **characterized in that** the biologically active moiety is in each case linked to the nanocomplex via a spacer.

8. Nanocomplexes or complex material according to any one of claims 1 to 7 for use in therapy.

9. Nanocomplexes or complex material according to any one of claims 1 to 7 for use in the controlled release of drugs after parenteral administration.

10. Nanocomplexes or complex material according to any one of claims 1 to 7 for use in the transport of drugs into cells.

11. Nanocomplexes or complex material according to any one of claims 1 to 7 for use in the transport of nucleic acids into cells.

12. Nanocomplexes or complex material according to any one of claims 8 to 11, wherein the use is directed towards at the therapy of a disease selected from monogenetic diseases, cancer, neurological diseases, vascular diseases, and infectuous diseases.

13. Method of formulating a drug, comprising the mixing of polyanions with peptide molecules with formation of the nanocomplexes according to any one of claims 1 to 5.

## Revendications

1. Nanocomplexes définis géométriquement, constitués d'un polyanion et de molécules peptidiques amphiphiles cationiques en un rapport équimolaire aux charges négatives présentes, **caractérisés en ce qu'**ils sont maintenus en cohésion par des interactions moléculaires coopératives et **en ce que** les molécules peptidiques comportent chacune 1 charge positive.

2. Nanocomplexes selon la revendication 1, **caractérisés en ce que** le polyanion, les molécules peptidiques, ou les deux, constituent un médicament pour une utilisation dans l'organisme humain ou animal.

3. Nanocomplexes selon la revendication 1 ou 2, **caractérisés en ce que** lés molécules peptidiques renferment chacune jusqu'à 20 acides aminés.

4. Nanocomplexes selon l'une des revendications 1 à 3, **caractérisés en ce que** le polyanion est choisi dans le groupe constitué des polysaccharides, des acides nucléiques et de leurs dérivés.

5. Nanocomplexes selon l'une des revendications 1 à 4, **caractérisés en ce qu'**ils présentent une forme linéaire.

6. Matière complexée, dans laquelle les nanocomplexes selon l'une des revendications 1 à 5 sont reliés à une unité biologiquement active pour l'identification sélective de tissus, de cellules ou d'organelles.

7. Matière complexée selon la revendication 6, **caractérisée en ce que** l'unité biologiquement active est à chaque fois reliée au nanocomplexe par le biais d'un espaceur.

8. Nanocomplexes ou matière complexée selon l'une des revendications 1 à 7 pour une utilisation en thérapie.

9. Nanocomplexes ou matière complexée selon l'une des revendications 1 à 7 pour une utilisation dans la libération contrôlée de médicaments après une application parentérale.

10. Nanocomplexes ou matière complexée selon l'une des revendications 1 à 7 pour une utilisation dans le transport de médicaments dans des cellules.

11. Nanocomplexes ou matière complexée selon l'une des revendications 1 à 7 pour une utilisation dans le transport d'acides nucléiques dans des cellules.

12. Nanocomplexes ou matière complexée selon l'une des revendications 8 à 11, dans lesquels l'utilisation est axée sur la thérapie d'une maladie choisie parmi les maladies monogéniques, le cancer, les maladies neurologiques, les maladies vasculaires et les maladies infectieuses.

13. Procédé de formulation d'un médicament qui comprend le mélange de polyanions avec des molécules peptidiques en formant des nanocomplexes selon l'une des revendications 1 à 5.
